# EUROPEAN PATENT APPLICATION

(11) **EP 4 821 454 A1**
(43) Date of publication of application: **14.10.2026**
(21) Application number: 24900866.5
(22) Date of filing: 04.11.2024
(51) Int. Cl.: A61B 34/10, A61B 90/50

(54) **VIRTUAL ENVIRONMENT-BASED INCISION SIMULATION MODELING DEVICE**

(30) Priority: 07.12.2023 KR 20230176175
(71) Applicant: Korea University Research and Business Foundation, Seoul 02841 (KR)
(72) Inventor: CHOI, June, Ansan-si Gyeonggi-do 15483 (KR); HWANG, In Tae, Ansan-si Gyeonggi-do 15355 (KR)
(74) Representative: IPAZ
(86) International application number: PCT/KR2024/017150
(87) International publication number: WO 2025/121679

(57) **Abstract**

The present specification discloses a virtual environment-based incision simulation modeling device. The incision simulation modeling device according to the present specification includes: a memory for storing modeling data for a virtual object that has three layers and modeling data for a virtual medical instrument; a virtual space provider for providing a virtual space in which the virtual object and the virtual medical instrument are arranged; a virtual medical instrument control unit for controlling the movement of the virtual medical instrument in the virtual space according to an external input signal; and a feedback controller for generating feedback on the virtual object or the virtual medical instrument when the virtual object and the virtual medical instrument come into contact with each other in the virtual space.

## Description

### [Technical Field]

The present invention relates to an incision simulation modeling device, and more particularly, to a virtual environment-based incision simulation modeling device.

### [Background Art]

The contents described in this part merely provide background information on the exemplary embodiment of the present specification, but do not constitute the related art.

In accordance with the development of the virtual environment technique, a medical surgery simulation technique in a virtual environment is being developed to improve a surgery proficiency of doctors who have less clinical experience. In order to enhance immersiveness of trainees in the medical surgery simulation, it is required to implement physical phenomena occurring during actual medical surgery to be similar to the reality in a virtual environment.

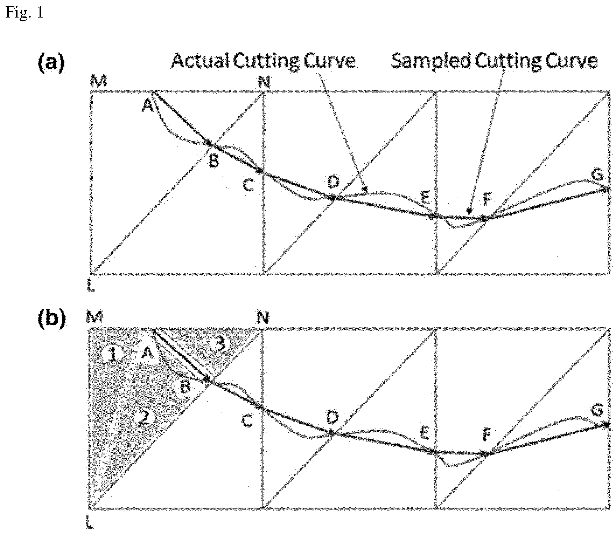

FIG. 1 illustrates a method of implementing incision of an affected area in a medical surgery simulation of the related art.

Referring to FIG. 1, a virtual three-dimensional affected object is implemented by a plurality of meshes formed by cloud points in the medical surgery simulation of the related art. In order to implement incision of the affected area of a patient which is performed in an actual medical operating room, a computer calculates an intersection between an actual cutting curve of a virtual medical instrument and a line which forms the mesh (FIG. 1A). Next, the computer calculates a sampled cutting curve obtained by connecting the intersections. Next, the computer performs a computational process of reconstructing the existing mesh shape to a plurality of sub meshes using the sampled cutting curve and each intersection.

A relatively large amount of computation is required for the medical surgery simulation of the related art to implement the incision of the patient's affected area. Accordingly, it takes a long time to render incision of the affected area in a virtual environment so that a response speed is slow and a cost for building and maintaining the infrastructure is increased.

### [Related Art Document]

### [Non-Patent Document]

(Non-Patent Document 1) Virtual Reality Simulator for Training in Myringotomy with Tube Placement, Caiwen Huang et al.. 2016, J. Med. Biol. Eng.

### [Disclosure]

### [Technical Problem]

An object of the present specification is to provide a virtual environment-based incision simulation modeling device.

Objects of the present specification are not limited to the above-mentioned objects, and other objects, which are not mentioned above, may be clearly understood by those skilled in the art from the following descriptions.

### [Technical Solution]

In order to achieve the above-described object, according to the present specification, an incision simulation modeling device may include a memory for storing modeling data for a virtual object that has three layers and modeling data for a virtual medical instrument; a virtual space provider for providing a virtual space in which the virtual object and the virtual medical instrument are arranged; a virtual medical instrument controller for controlling the movement of the virtual medical instrument in the virtual space according to an external input signal; and a feedback controller for generating a feedback on the virtual object or the virtual medical instrument when the virtual object and the virtual medical instrument come into contact with each other in the virtual space.

According to the exemplary embodiment of the present specification, three layers of the virtual object may include: a first layer which forms an incision target; a second layer which forms a non-incision target; and a third layer for generating a vertical repulsive force of the virtual medical instrument.

At this time, the second layer is disposed between the first layer and the third layer and an area of the second layer corresponding to the first layer may be formed as a blank area.

According to the exemplary embodiment of the present specification, the first layer has a mesh shape formed by cloud points and the feedback controller may eliminate a mesh in an area of the first layer which is in contact with the virtual medical instrument.

According to the exemplary embodiment of the present specification, the feedback controller may generate a feedback signal for a movement resistance of the virtual medical instrument when the virtual medical instrument comes into contact with the first layer.

According to the exemplary embodiment of the present specification, the feedback controller may move a position of the cloud point adjacent to the mesh of the first layer which is in contact with the virtual medical instrument to be eliminated to a direction in which the virtual medical instrument moves by a predetermined amount.

According to the exemplary embodiment of the present specification, the feedback controller models outflow of a body fluid when the mesh is eliminated to generate feedback.

According to the exemplary embodiment of the present specification, the feedback controller may generate a feedback signal which limits entry of the virtual medical instrument when the virtual medical instrument comes into contact with the third layer.

The incision simulation modeling device according to the present specification may be one component of an incision simulation system including: a virtual medical instrument control device which outputs a movement signal of the virtual medical instrument to a virtual medical instrument controller of the incision simulation modeling device.

According to the exemplary embodiment of the present specification, the virtual medical instrument control device may include: an incision signal generator which outputs an incision signal to the incision simulation modeling device.

According to the exemplary embodiment of the present specification, the virtual medical instrument control device may include: a haptic provider which provides haptic reaction to a user by a signal output by a feedback controller of the incision simulation modeling device.

The incision simulation system according to the present specification may further include: a display device which displays a virtual space provided by a virtual space provider of the incision simulation modeling device, the virtual object, and a virtual medical instrument.

According to the exemplary embodiment of the present specification, the display device may be a predetermined shape of display mounted on a head of a user.

Other detailed matters of the present invention are included in the detailed description and the drawings.

### [Advantageous Effects]

According to one aspect of the present specification, only an area of a first layer which forms an incision target is implemented by meshes formed by cloud points to model the virtual object using a relatively less memory than that of the related art.

According to another aspect of the present specification, a mesh of an area which is in contact with a virtual medical instrument is eliminated to model the incision simulation with a relatively less computational amount than that of the related art.

According to still another aspect of the present specification, the incision simulation is modeled with a less computational amount than that of the related art so that a simulation response speed is relatively faster than that of the related art.

According to still another aspect of the present specification, the incision simulation is modeled with a less computational amount than that of the related art so that relatively less cost is used for building, using and/or maintaining infrastructures.

The effects of the present invention are not limited to the technical effects mentioned above, and other effects which are not mentioned can be clearly understood by those skilled in the art from the following description

### [Description of Drawings]

FIG. 1 illustrates a method of implementing incision of an affected area in a medical surgery simulation of the related art.
FIG. 2 is a block diagram of an incision simulation modeling device according to an exemplary embodiment of the present specification.
FIG. 3 illustrates a human tympanic membrane image.
FIG. 4 is an exemplary image of modeling data of a virtual object loaded by a modeling program.
FIG. 5 is an exemplary image of incision simulation modeling according to the present specification.
FIG. 6 is another exemplary image of incision simulation modeling according to the present specification.
FIG. 7 is still another exemplary image of incision simulation modeling according to the present specification.
FIG. 8 is a block diagram of an incision simulation system according to an exemplary embodiment of the present specification.
FIG. 9 is a block diagram of an incision simulation system according to another exemplary embodiment of the present specification.

### [Best Mode]

Advantages and characteristics of the present invention disclosed in the present specification, and a method of achieving the advantages and characteristics will be clear by referring to exemplary embodiments described below in detail together with the accompanying drawings. However, the present specification is not limited to exemplary embodiments disclosed herein but will be implemented in various different forms. The exemplary embodiments are provided by way of example only so that a person of ordinary skilled in the art may fully understand the disclosures of the present specification and the scope of the present specification. Therefore, the present specification will be defined only by the scope of the appended claims.

The terms used in the present specification are for explaining the embodiments rather than limiting the scope of the present specification. Unless particularly stated otherwise in the present specification, a singular form also includes a plural form. The term "comprise" and/or "comprising" used in the specification does not exclude the presence or addition of one or more other components in addition to the mentioned component.

Like reference numerals generally denote like elements throughout the specification and "and/or" includes each of mentioned components and all combinations of one or more components. Although the terms "first", "second", and the like are used for describing various components, these components are not confined by these terms. These terms are merely used for distinguishing one component from the other components. Therefore, a first component to be mentioned below may be a second component in a technical spirit of the present invention.

Unless otherwise defined, all terms (including technical and scientific terms) used in the present specification may be used as the meaning which may be commonly understood by the person with ordinary skill in the art, to which the present invention belongs. It will be further understood that terms defined in commonly used dictionaries should not be interpreted in an idealized or excessive sense unless expressly and specifically defined.

Spatially relative terms such as "below", "beneath", "lower", "above", or "upper" may be used to easily describe the correlation between one component and other components as illustrated in the drawings. The spatially relative terms should be understood to include different directions of the elements when the elements are used or operated in addition to the direction illustrated in the drawings. For example, when the component illustrated in the drawing is turned over, a component which is disposed below or beneath other component may be disposed above the other component. Accordingly, the exemplarily term "below" or "beneath" may include both downward and upward directions. The components may be oriented in different directions so that terms which are spatially relative may be interpreted according to the orientation.

Hereinafter, exemplary embodiments of the present invention will be described in detail with reference to the accompanying drawings.

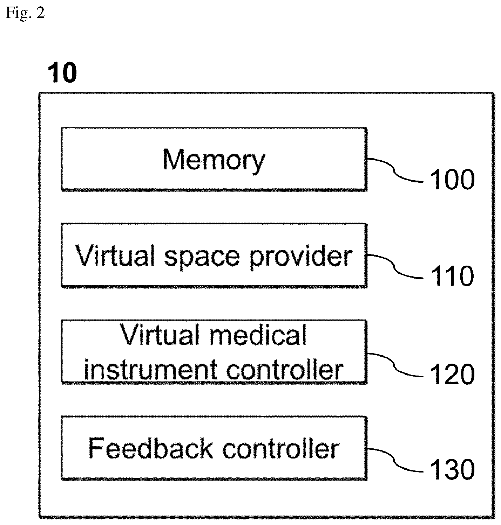

FIG. 2 is a block diagram of an incision simulation modeling device according to an exemplary embodiment of the present disclosure.

Referring to FIG. 2, an incision simulation modeling device 10 according to an exemplary embodiment of the present specification may include a memory 100, a virtual space provider 110, a virtual medical instrument controller 120, and a feedback controller 130.

The memory 100 may store modeling data for a virtual object having three layers and model data for a virtual medical instrument. The virtual object may correspond to a virtual affected area which is modeled for incision simulation. Further, the virtual object may correspond to a virtual human structure including a specific affected area which is modeled for an incision simulation.

According to an exemplary embodiment of the present specification, the virtual object may correspond to a virtual tympanic membrane modeled for myringotomy simulation. Further, the virtual object may correspond to a virtual human structure including tympanic membrane and middle ear areas which are modeled for myringotomy simulation. Further, the virtual object may correspond to a virtual human structure including tympanic membrane, external auditory canal and/or middle ear areas which are modeled for myringotomy simulation. This corresponds to an example and the virtual object may vary depending on a type of an incision simulation to be implemented in the virtual environment. Further, the memory 100 may store modeling data for at least one virtual object for at least one incision simulation.

The virtual medical instrument may correspond to a virtual myringotomy knife for incision of the virtual tympanic membrane. This corresponds to an example and the virtual medical instrument may vary depending on a type of a surgery incision simulation to be implemented in the virtual environment. Further, the memory 100 may store modeling data for at least one virtual medical instrument for at least one incision simulation.

According to the exemplary embodiment of the present specification, three layers of the virtual object may include a first layer which forms an incision target, a second layer which forms a non-incision target, and a third layer for generating a vertical repulsive force of the virtual medical instrument.

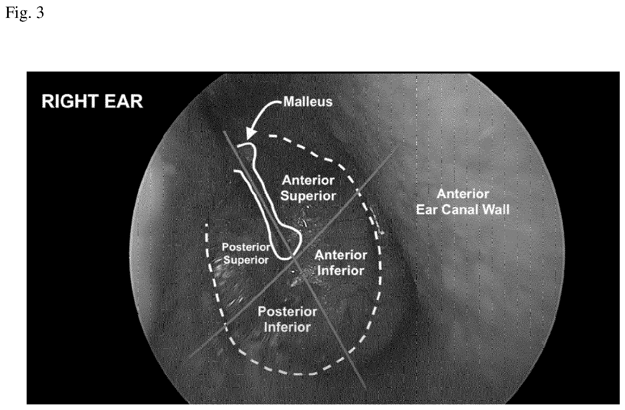

FIG. 3 illustrates a human tympanic membrane image.

Referring to FIG. 3, the tympanic membrane is divided into anterior superior and posterior superior areas which are in contact with malleus and anterior inferior and posterior inferior areas which are not in contact with malleus. When actual myringotomy is performed, the anterior inferior and posterior inferior areas are incised to drain the effusion. The "anterior" is a direction toward the human face and occipital area, the "superior" is a direction toward the crown of the head, and "inferior" is a direction toward the leg.

In the modeling data of the virtual object stored in the memory 100 for the myringotomy simulation, modeling data for the first layer may correspond to modeling data for the anterior inferior and posterior inferior areas of the tympanic membrane. The modeling data for the second layer may correspond to modeling data for the anterior superior and posterior superior areas of the tympanic membrane including malleus. Further, the modeling data for the second layer may further include modeling data for a part of the external auditory canal. The modeling data for the third layer may correspond to modeling data for a part of a medial wall of the middle ear.

The virtual space provider 110 may provide a virtual space in which the virtual object and the virtual medical instrument are disposed. The memory 100 may further include modeling data for the virtual space. The virtual space provider 110 may load the modeling data of the virtual space from the memory 100. The virtual space provider 110 may render the virtual space using the modeling data of the virtual space.

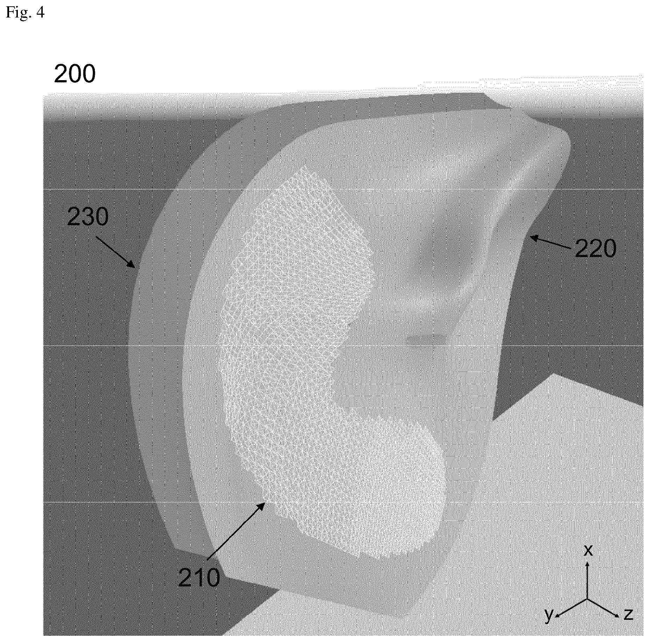

FIG. 4 is an exemplary image of modeling data of a virtual object loaded by a modeling program.

Referring to FIG. 4, the modeling data of the virtual object 200 stored in the memory 100 may include the first layer 210, the second layer 220, and the third layer 230. The second layer 220 may be disposed between the first layer 210 and the third layer 230. It may mean that the first layer 210, the second layer 220, and the third layer 230 are laminated.

The first layer 210 may have a relatively largest z-axis coordinate value on the virtual space. The second layer 220 may have a second largest z-axis coordinate value next to the first layer 210 on the virtual space. The third layer 230 may have a relatively smallest z-axis coordinate value on the virtual space. It may mean that the first layer 210, the second layer 220, and the third layer 230 are laminated on the z-axis of the virtual space.

The difference between the z-axis coordinate value of the first layer 210 and the z-axis coordinate value of the second layer 220 may be set within a predetermined difference. The difference between the z-axis coordinate value of the second layer 220 and the z-axis coordinate value of the third layer 230 may be set within a predetermined difference.

The coordinate axis on which the first layer 210, the second layer 220, and the third layer 230 are laminated on the virtual space varies depending on the alignment of the virtual object 200, but is not limited by a specific coordinate axis.

The first layer 210 may be overlaid on the second layer 220. A region of the second layer 220 in which the first layer 210 overlaps may be formed by a blank area.

A shape of the blank area may be the same as the shape of the first layer 210. Further, the shape of the blank area may be larger than the shape of the first layer 210 by a predetermined size. Further, the shape of the blank area may be smaller than the shape of the first layer 210 by a predetermined size.

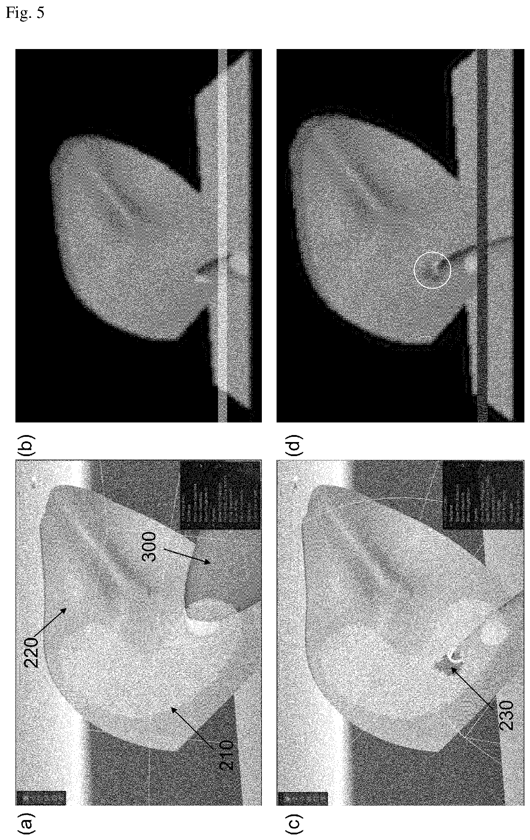

FIG. 5 is an exemplary image of incision simulation modeling according to the present specification.

Referring to FIG. 5, in the virtual space, the virtual object 200 and the virtual medical instrument 300 may be disposed.

The virtual medical instrument controller 120 may control a movement of the virtual medical instrument 300 in the virtual space according to an external input signal.

The feedback controller 130 may generate feedback on the virtual object 200 and/or the virtual medical instrument 300 when the virtual object 200 and the virtual medical instrument 300 come into contact with each other in the virtual space.

The feedback controller 130 may determine whether the virtual object 200 and the virtual medical instrument 300 come into contact with each other in the virtual space. If the feedback controller 130 may determine that the virtual object 200 and the virtual medical instrument 300 come into contact with each other, the feedback controller 130 may generate a visual feedback which deforms the shape of the virtual object 200. Further, the feedback controller 130 may generate a tactile feedback signal corresponding to a force generated when a human tissue is incised by an actual medical instrument.

According to the exemplary embodiment of the present specification, the feedback controller 130 may determine whether the virtual medical instrument 300 is in contact with the first layer 210. If the feedback controller 130 determines that the first layer 210 and the virtual medical instrument 300 come into contact with each other, the feedback controller 130 may generate a visual feedback to the first layer 210. The visual feedback generated in the virtual first layer 210 means that incision of the human tissue is implemented during the actual medical surgery process.

In order to implement the incision of the human tissue, the modeling data of the first layer 210 may correspond to modeling data which creates the first layer 210 as a cloud point based mesh structure in the virtual space (FIG. 5A). The modeling data of the second layer 220 and the third layer 230 may correspond to surface modeling data. This corresponds to an example, but is not limited by the modeling method. Only the first layer 210 is created as the mesh structure so that the incision simulation modeling device 10 according to the present specification may model a virtual object using a relatively less memory than the incision simulation modeling device of the related art.

Referring to FIG. 5A, if the feedback controller 130 determines that the first layer 210 and the virtual medical instrument 300 do not come into contact with each other, the feedback controller 130 does not generate a visual feedback to the first layer 210.

Referring to FIG. 5B, it is confirmed that when the virtual object 200 is rendered, the visual feedback by the feedback controller 130 is not generated in an area corresponding to the first layer 210.

Referring to FIG. 5C, if the feedback controller 130 determines that the first layer 210 and the virtual medical instrument 300 come into contact with each other, the feedback controller 130 may generate a visual feedback to the first layer 210. The feedback controller 130 may generate the visual feedback by removing the area of the first layer 210 which is in contact with the virtual medical instrument 300.

The feedback controller 130 may generate information about a coordinate value of an area where the first layer 210 and the virtual medical instrument 300 come into contact with each other. The feedback controller 130 may eliminate the mesh of the first layer 210 including the coordinate value of the area by generating the visual feedback. This means that the feedback controller 130 generates the visual feedback to delete data which generates the mesh including the coordinate value of the area in the virtual space. Alternatively, this means that the feedback controller 130 generates the visual feedback to delete data of points which configure the mesh including the coordinate value of the area.

Referring to FIG. 5D, when the virtual object 200 is rendered, an area of the first layer 210 which is in contact with the virtual medical instrument 300 may be removed by the visual feedback generated by the feedback controller 130.

In order to determine whether the first layer 210 and the virtual medical instrument 300 come into contact with each other, bounding box collision detection, separating axis theorem, bounding volume hierarchy, or Gilbert-Johnson-Keerthi algorithm is used, and this corresponds to an example, but is not limited by a specific algorithm.

The incision simulation modeling device 10 according to the present specification removes the mesh of the first layer 210 from the area which is in contact with the virtual medical instrument 300 to omit a computational process of rebuilding a mesh to sub meshes in the incision simulation device of the related art, to perform relatively less operation.

If the area of the first layer 210 which is in contact with the virtual medical instrument 300 is removed, modeling is performed to show an area of the third layer 230 corresponding to the removed area. At this time, the third layer 230 may be modeled with a relatively darker color value than those of the first layer 210 and the second layer 220. Alternatively, the third layer 230 may be modeled with a color value different from those of the first layer 210 and the second layer 220. Alternatively, the third layer 230 may be modeled to include a predetermined shape of marker. This corresponds to an example, but is not limited by the above-described method.

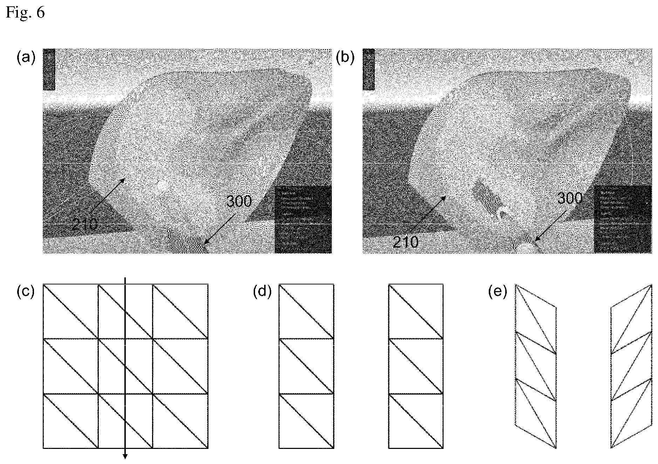

FIG. 6 is another exemplary image of incision simulation modeling according to the present specification.

Referring to FIGS. 6A and 6B, the virtual medical instrument 300 may move to a right lower end while maintaining a contact state with the first layer 210. The feedback controller 130 may eliminate the mesh of the first layer 210 which comes into contact while the virtual medical instrument 300 moves.

Further, the feedback controller 130 deforms the mesh of the first layer 210 toward a movement direction of the virtual medical instrument 300. The feedback controller 130 may move a position of cloud points which are adjacent to the mesh which is in contact with the virtual medical instrument 300 and eliminated to the movement direction of the virtual medical instrument 300 by a predetermined amount.

For example, the virtual medical instrument 300 may move downwardly while maintaining the contact state with the first layer 210 (see FIG. 6C). The feedback controller 130 may eliminate the mesh of the first layer 210 which comes into contact with the virtual medical instrument 300 (see FIG. 6D). The feedback controller 130 may select a cloud point adjacent to the eliminated mesh in FIG. 6D. Next, the feedback controller 130 may move the cloud point to a movement direction of the virtual medical instrument 300 by a predetermined amount. By using this, the feedback controller 130 may implement in the virtual object 200 that the incised surface of the human tissue is dragged to the movement direction of the surgical instrument due to a frictional force between the surgical instrument and the incised surface of the human tissue during the actual incision surgery. This corresponds to an example and is not limited by a specific movement direction of the virtual medical instrument 300.

In FIG. 6B, when the virtual medical instrument 300 moves to the right lower end while maintaining the contact state with the first layer 210, the feedback controller 130 may generate a tactile feedback signal for a movement resistance of the virtual medical instrument 300. The movement resistance refers to a reaction force of the surgical instrument received from the human tissue located in the movement direction during the actual incision surgery. Further, the movement resistance may correspond to a frictional force of the surgical instrument received from the incised surface of the human tissue during the actual incision surgery. Further, the movement resistance may refer to a net force of the reaction force and the frictional force. Further, the movement resistance may refer to a net force for all forces acting in an opposite direction of the movement direction of the surgical instrument due to interaction of the surgical instrument and the human tissue. Referring to FIG. 6B, the feedback controller 130 may generate a tactile feedback signal for the movement resistance acting in a left upper end which is opposite to the movement direction of the virtual medical instrument 300. This corresponds to an example, but is not limited by a specific direction.

The feedback controller 130 may generate a visual feedback by modeling outflow of body fluid when the mesh of the first layer 210 is eliminated. The memory 100 may further include body fluid outflow modeling data. The feedback controller 130 may fetch the body fluid outflow modeling data from the memory 100. The feedback controller 130 may model body fluid outflow in an area in which the mesh is eliminated when the mesh of the first layer 210 is eliminated. In the tympanic membrane incision simulation, the body fluid outflow modeling data may correspond to data of modeling outflow of the effusion. This corresponds to an example, but is not limited by the body fluid.

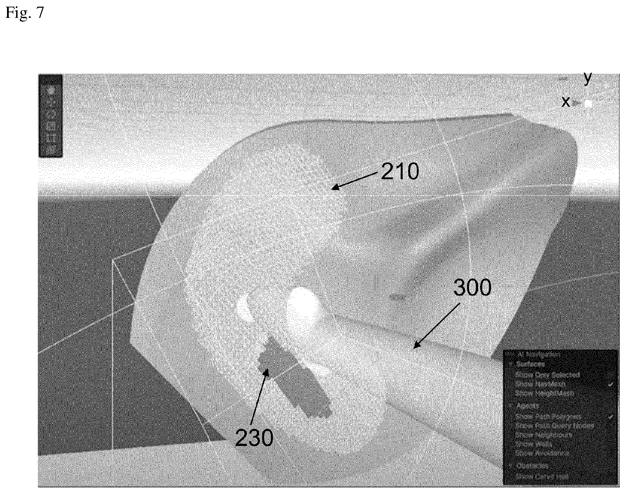

FIG. 7 is still another exemplary image of incision simulation modeling according to the present specification.

Referring to FIG. 7, the virtual medical instrument 300 passes through the blank area between the first layer 210 and the third layer 230 to be in contact with the third layer 230. When the virtual medical instrument 300 is in contact with the third layer 230, the feedback controller 130 may generate a tactile feedback signal which limits entry of the virtual medical instrument 300. The feedback signal may be a signal corresponding to a vertical reaction force acting in an opposite direction to the entering direction of the virtual medical instrument 300.

The entering direction of the virtual medical instrument 300 may refer to a vertical direction which is directed to the third layer 230 from the first layer 210.

In the virtual space, the first layer 210 may have a z-axis coordinate value relatively larger than that of the third layer 230. The entering direction of the virtual medical instrument 300 may be a direction in which the z-axis coordinate value is relatively reduced.

When an actual myringotomy is performed, there may be a situation in which the surgical instrument incises the tympanic membrane and comes into contact with an inner wall of the middle ear. At this time, a vertical repulsive force may act from the inner wall of the middle ear to the surgical instrument.

When the virtual medical instrument 300 is in contact with the third layer 230 in the myringotomy simulation, the feedback controller 130 may output a tactile feedback signal corresponding to the vertical repulsive force generated during the actual surgery to the virtual medical instrument 300.

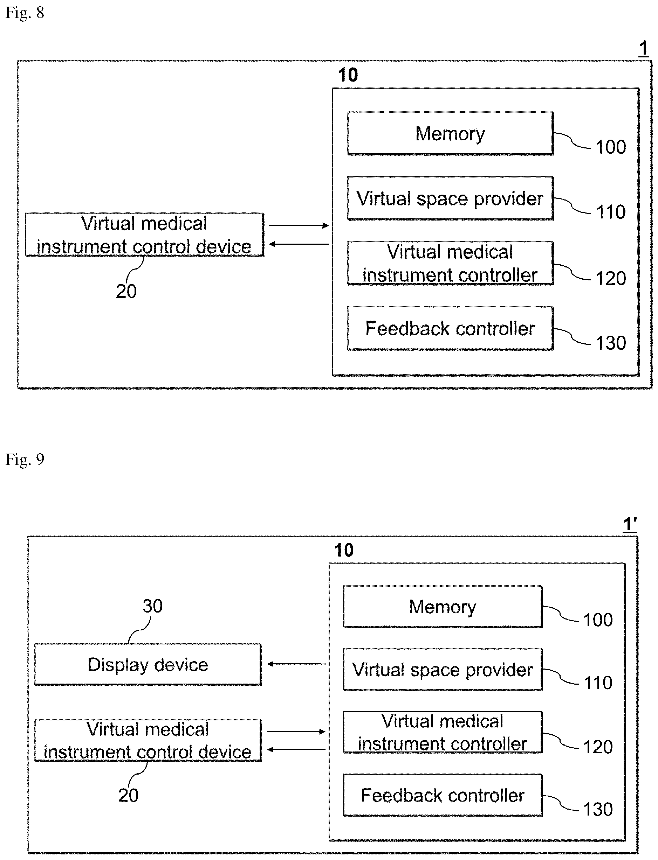

FIG. 8 is a block diagram of an incision simulation system according to an exemplary embodiment of the present specification.

Referring to FIG. 8, an incision simulation system 1 according to an exemplary embodiment of the present specification may include the incision simulation modeling device 10 and a virtual medical instrument control device 20. The virtual medical instrument control device 20 may output a signal according to the movement of a user to the virtual medical instrument controller 120. The virtual medical instrument controller 120 receives the movement signal to control a movement of the virtual medical instrument 300. The virtual medical instrument control device 20 may be a device having the same shape as a pen shape device connected to a plurality of robot arms or an actual surgical instrument, and this corresponds to an example, but is not limited by a specific shape.

The virtual medical instrument control device 20 may include an incision signal generator which outputs an incision signal to the incision simulation modeling device 10. The incision signal generator may correspond to a predetermined shape of button or touch sensor and this corresponds to an example, but is not limited by a specific device.

When the virtual medical instrument 300 is in contact with the first layer 210, the user may generate an incision signal using the incision signal generator. The incision signal may correspond to a signal which allows the feedback controller 130 to remove a mesh of an area where the virtual medical instrument 300 and the first layer 210 come into contact with each other.

The virtual medical instrument control device 20 may include a haptic provider which provides a haptic response to the user by a signal output from the feedback controller 130. The signal output from the feedback controller 130 may correspond to a signal corresponding to the movement resistance and/or the vertical repulsive force. The virtual medical instrument control device 20 provides the haptic response to the movement resistance and/or the vertical repulsive force to the user to relatively increase user's simulation immersiveness.

FIG. 9 is a block diagram of an incision simulation system according to another exemplary embodiment of the present specification.

Referring to FIG. 9, an incision simulation system 1' according to another exemplary embodiment of the present specification may include an incision simulation modeling device 10, a virtual medical instrument control device 20, and a display device 30.

The display device 30 may display a virtual space provided by the virtual space provider 110, the virtual object 200, and the virtual medical instrument 300. The display device 30 may be a predetermined shape of display which is mounted on a head of the user. For example, the display device may correspond to smart glasses, smart goggle, head mounted display, or helmet mounted display of virtual reality (VR) equipment and/or augmented reality (AR) equipment. This corresponds to an example and the display device may include various devices, such as cathode-ray tube (CRT), plasma display panel (PDP), liquid crystal display (LCD), light emitting diode (LED), or organic light emitting diode (OLED), and is not limited by a specific display device.

The memory 100, the virtual space provider 110, the virtual medical instrument controller 120, and the feedback controller 130 include processors, application-specific integrated circuits (ASIC), other chipsets, logic circuits, registers, communication modems, and data processing devices which are known in the technical field to which the present invention belongs to execute the calculation and various control logics. Further, when the above-described control logic is implemented by software, the memory 100, the virtual space provider 110, the virtual medical instrument controller 120, and the feedback controller 130 are implemented as a set of program modules. At this time, the program module is stored in the memory device and is executed by the processor.

In the present specification, description was modeled by assuming a simulation situation of the myringotomy. This corresponds to an example, and modeling data for the virtual object and the virtual medical instrument may vary depending on various incision simulations, such as pericardiotomy and/or durotomy and the generated feedback also varies. Accordingly, the present specification is not limited to the myringotomy simulation and there may be various exemplary embodiments depending on the type of the incision simulation.

The above-described exemplary embodiments of the present specification have been described with reference to the accompanying drawings, but those skilled in the art will understand that the present specification may be implemented in another specific form without changing the technical spirit or an essential feature thereof. Therefore, it should be understood that the above-described exemplary embodiments are illustrative in all aspects and do not limit the present specification.

### [Explanation of Reference Numerals]

1: Incision simulation system
10: Incision simulation modeling device
20: Virtual medical instrument control device
30: Display device
100: Memory
110: Virtual space provider
120: Virtual medical instrument controller
130: Feedback controller

## Claims

1. An incision simulation modeling device, comprising:
a memory for storing modeling data for a virtual object that has three layers and modeling data for a virtual medical instrument;
a virtual space provider for providing a virtual space in which the virtual object and the virtual medical instrument are arranged;
a virtual medical instrument controller for controlling the movement of the virtual medical instrument in the virtual space according to an external input signal; and
a feedback controller for generating feedback on the virtual object or the virtual medical instrument when the virtual object and the virtual medical instrument come into contact with each other in the virtual space.

2. The incision simulation modeling device of claim 1, wherein three layers of the virtual object include:
a first layer which forms an incision target;
a second layer which forms a non-incision target; and
a third layer for generating a vertical repulsive force of the virtual medical instrument.

3. The incision simulation modeling device of claim 2, wherein the second layer is disposed between the first layer and the third layer and an area of the second layer corresponding to the first layer is formed as a blank area.

4. The incision simulation modeling device of claim 2, wherein the first layer has a mesh shape formed by cloud points and the feedback controller eliminates a mesh in an area of the first layer which is in contact with the virtual medical instrument.

5. The incision simulation modeling device of claim 4, wherein the feedback controller moves a position of the cloud point adjacent to the mesh of the area of the first layer which comes into contact with the virtual medical instrument to be eliminated to a direction in which the virtual medical instrument moves by a predetermined amount.

6. The incision simulation modeling device of claim 4, wherein the feedback controller generates a feedback signal for a movement resistance of the virtual medical instrument when the virtual medical instrument comes into contact with the first layer.

7. The incision simulation modeling device of claim 4, wherein the feedback controller models outflow of a body fluid when the mesh is eliminated to generate a feedback.

8. The incision simulation modeling device of claim 8, wherein the feedback controller generates a feedback signal to limit the entry of the virtual medical instrument when the virtual medical instrument comes into contact with the third layer.

9. An incision simulation system, comprising:
an incision simulation modeling device of any one of claims 1 to 8; and
a virtual medical instrument control device which outputs a movement signal of a virtual medical instrument to a virtual medical instrument controller of the incision simulation modeling device.

10. The incision simulation system of claim 9, wherein the virtual medical instrument control device includes:
an incision signal generator which outputs an incision signal to the incision simulation modeling device.

11. The incision simulation system of claim 9, wherein the virtual medical instrument control device includes:
a haptic provider which provides haptic reaction to a user by a signal output by a feedback controller of the incision simulation modeling device.

12. The incision simulation system of claim 9, further comprising:
a display device which displays a virtual space provided by a virtual space provider of the incision simulation modeling device, the virtual object, and a virtual medical instrument.

13. The incision simulation system of claim 12, wherein the display device is a predetermined shape of display which is mounted on a head of a user.
